# EUROPEAN PATENT APPLICATION

(11) **EP 4 760 740 A1**
(43) Date of publication of application: **17.06.2026**
(21) Application number: 24219440.5
(22) Date of filing: 12.12.2024
(51) Int. Cl.: G16H 40/40, A61N 1/372, G16H 40/67

(54) **REMOTE PROGRAMMING ASSEMBLY ENABLING SAFE COMMUNICATION BETWEEN A PROGRAMMING DEVICE AND AN IMPLANTABLE MEDICAL DEVICE**

(71) Applicant: BIOTRONIK SE & Co. KG, 12359 Berlin (DE)
(72) Inventor: MUESSIG, Dirk, West Linn, OR 97068 (US); FRYER, Alan, Portland, OR 97202 (US); WHITTINGTON, R. Hollis, Portland, OR 97202 (US); VON ARX, Jeffrey A., Lake Oswego, OR 97035 (US)
(74) Representative: Biotronik Corporate Services SE

(57) **Abstract**

The invention relates to a remote programming assembly (100), comprising: an implantable medical device (101); a programming device (103) located remote from the implantable medical device (101); and a patient device (102) operatively connected to the implantable medical device (101) and to the programming device (103). The patient device (102) and the implantable medical device (101) are designed and configured to communicate via a near-field communication with each other. In addition, the patient device (102) and the implantable medical device (101) are designed and configured to allow a reprogramming of operational parameters of the implantable medical device (101) only upon activation of a physical user interface element (1023) of the patient device (102) by a user.

## Description

The instant disclosure generally relates to a remote programming assembly enabling safe communication between a programming device and an implantable medical device.

Remote interrogation of an implantable medical device using an inductive communications link of the implantable medical device (normally used to interface with a clinician programmer) can be generally used by a patient device to obtain the same information that the programmer obtains during a normal in-clinic follow-up. In addition, remote interrogation can be used and carried out by a service center (e.g. Home Monitoring Service Center) in order to be able to ensure comprehensive care for the patient.

In addition to remote interrogation, remote programming of medical devices is a very interesting functionality. However, remote programming of medical devices has been in discussion for over a decade with very little progress due to concerns about cybersecurity. With the move to telehealth motivated by the Covid pandemia, remote work and remote care continue to become even more important.

The biggest drawback of the implantable medical devices already available on the market is that in case a reprogramming of the implantable medical devices is required, the patient must be brought into a clinic. This requires additional workload for the medical staff, e.g., for scheduling the patients, as well as for the patient because of, e.g., the travel requirements to a follow-up clinic. Another drawback of the known solutions is the missing re-programmability of the implantable medical device from the remote server where it is for technical or energetic reasons not possible to have an end-to-end encryption from the implant to the remote server.

Some implantable medical devices (implants) known from prior art have radiofrequency (RF) solutions for home monitoring allowing an RF-based remote programing. Such RF-based solutions typically feature an end-to-end connection to the implant via a patient device that relays the programming commands.

Some patient devices known from prior have only a coil communication interface for communicating to an implant. In this case, typically the coil communication link is primarily protected by proximity and not by cryptography.

Fundamentally, there are concerns about security of remote programming in multiple areas. Examples include:
- A threat actor gaining access to manufacturer's credentials and doing a remote programming on multiple implantable medical devices maliciously.
- A threat actor directly attacking parts of the communication system to achieve the same ends without valid credentials.

Particularly, there are concerns of directly attacking a patient device which is likely in the patient's home. The patient device could be attacked physically, or it could be attacked remotely over its exposed interfaces. In particular, it could be attacked over its interfaces that allow software updates which are critical for software maintenance.

It is an object of the present invention to provide a novel solution to allow remote programming of an implantable medical device by a remote server to in a secure way, wherein the solution provides an enhanced protection against remote attacks, such as cyberattacks, on the implantable medical device.

This object is achieved with a remote programming assembly having the claim elements explained in the following. Such a remote programming assembly comprises an implantable medical device, a programming device that is located remote from the implantable medical device, and a patient device that is operatively connected to the implantable medical device and to the programming device. The patient device and the implantable medical device are constructed such as to communicate with each other via a near-field communication link. Furthermore, the patient device and the implantable medical device are constructed such as to allow a reprogramming of operational parameters of the implantable medical device only upon activation of a physical user interface element of the patient device by a user.

Expressed in other words, the implantable medical device that forms part of the claimed remote programming assembly is capable of being interrogated by a patient device, which is not the traditional physician programmer. Rather, the patient device is able to send information back to an off-site server (i.e., the programming device) to provide information on the implantable medical device and on the patient status. In addition, the patient device is able to send programming commands to the implantable medical device. However, this is only possible after the user of the patient device has performed a physical interaction with the patient device. Thus, the present invention provides different levels of access to the implantable medical device via the patient device and enables to temporarily give greater access to the implantable medical device in a home environment to support remote programming of the implantable medical device. The security of such communication between the patient device and the implantable medical device allowing remote programming is subject to a physical interaction of the user with the patient device. Due to this necessity of physical interaction, it will not be possible to compromise the patient device via a remote attack or cyber-attack. Rather, the possibility of allowing reprogramming of the implantable medical device will only be given after a physical interaction between the user and the patient device has been taken place. This significantly increases the security of the presently described remote programming approach, while generally enabling such remote programming.

In an embodiment, the programming device is a cloud-based user interface allowing clinicians to perform remote programming on the implantable medical device. In an embodiment, the programming device comprises a business layer that turns the user input into commands/new parameters and sends them to the patient device associated with the implantable medical device (briefly also referred to as implant).

In an embodiment, the activation of the physical user interface element is realized by an intentional interaction of the user with the physical user interface element. In an embodiment, the activation of the physical user interface element is realized by an interaction, e.g. a haptic interaction, between the user and the physical user interface element. Particularly appropriate examples of such interaction for achieving an activation of the physical user interface element are the reading-out of a fingerprint of the user, the entering of an authentication key by the user (such as a personal identification number (PIN) or a specific pattern to be drawn by the user on a touch sensitive screen of the patient device), the facial recognition of the user by a camera of the patient device, the pushing of a button on the patient device, and the performance of a predeterminable physical movement of the patient device. To give an example, it may be required that the patient moves the patient device three times up and down and two times from left to right to activate the reprogramming function of the patient device. Obviously, completely other movement patterns could also be required to activate the reprogramming functionality of the patient device. The patient device can generally have an appearance like a smart phone, wherein the patient device does not require all functions of a smart phone (but requires the functions as described herein).

In an embodiment, the patient device is constructed such to allow the reprogramming or the start of the reprogramming of the operational parameters of the implantable medical device only during a predeterminable period of time upon activation of the physical user interface element of the patient device. This predeterminable period of time lies, in an embodiment, with in a range of 1 minute to 10 minutes, in particular 2 minutes to 9 minutes, in particular 3 minutes to 8 minutes, in particular 4 minutes to 7 minutes, in particular 5 minutes to 6 minutes. If no reprogramming commands are received by the patient device during this predeterminable period of time, the enablement of reprogramming the implantable medical device is deactivated again. Such a time-out functionality secures the integrity of the implantable medical device, e.g., in case that there are transmission errors between the remote programming device and the implantable medical device.

In an embodiment, the patient device is designed and configured to no longer allow the reprogramming of the operational parameters of the implantable medical device once a termination command is received by the patient device. Then, it will be necessary to activate the physical user interface element once again to transfer the patient device into a state in which it allows the reprogramming of the operational parameters of the implantable medical device.

In an embodiment, the patient device is constructed such to generate an authentication code and to forward a reprogramming command received by the patient device from the programming device to the implantable medical device only if the reprogramming command comprises the authentication code. Thus, the authentication code additionally secures the integrity of the data transfer between the programming device and the patient device. The authentication code can be or generated from a unique pre-shared key that is valid per patient device. In an embodiment, the authentication code is a one-use code that is newly generated each time a reprogramming of the implantable medical device is intended. In an embodiment, a session-based concept akin to Transport Layer Security (TLS) is applied in which authentication, key negotiation and session-key based encryption/authentication is done based on a public/private key system. In this embodiment, such a public/private key pair is generated in a trusted platform module on the patient device. The trusted platform module also serves for storing the public/private key pair so that the private key is never shared out of the patient device. This enables a particularly high encryption standard for the data communication between the programming device and the patient device.

To put it somewhat more abstractly, the reprogramming command comprises, in an embodiment, the authentication code in a cryptographically secured manner. This makes it more difficult to intercept the authentication code and to perform a cyberattack onto the communication link between the programming device and the patient device.

In an embodiment, the authentication code has a limited validity period, such as, e.g., 1 minute to 1 hour, in particular 2 minutes to 50 minutes, in particular 3 minutes to 45 minutes, in particular 4 minutes to 40 minutes, in particular 5 minutes to 35 minutes, in particular 6 minutes to 30 minutes, in particular 7 minutes to 25 minutes, in particular 8 minutes to 20 minutes, in particular 9 minutes to 15 minutes, in particular 10 minutes to 12 minutes. Such a limited validity period also increases the security of the communication link between the programming device and the patient device since an interception of the authentication code will not be useful for a threat actor after expiration of the validity period.

In an embodiment, the patient device is constructed such that it requires a multiple-step activation (comprising two or more steps) of the physical user interface element of the patient device. In this context, a first step of the multiple-step activation serves for generally initiating a reprogramming procedure for the reprogramming of operational parameters of the implantable medical device. In addition, a second or subsequent step serves for confirming the specific intended reprogramming command as part of the reprogramming procedure. While generally a single-step activation process of the reprogramming functionality of the patient device is conceivable, this multiple-step activation can enhance the safety of the whole reprogramming functionality. The additionally required acceptance of specific remote programming commands by the second or subsequent step can be used for confirming that the intended changes of the operational parameters of the implantable medical device indeed make sense. This can be realized, e.g., by an additional dialogue with the remote clinician operating the programming device.

In an embodiment, the patient device comprises a first portion and a second portion. The first portion is an updatable portion, wherein the second portion is a secured non-updatable portion. In this context, the second portion serves for establishing a communication link to the implantable medical device for allowing the reprogramming of operational parameters of the implantable medical device upon activation of the physical user interface element of the patient device. In an embodiment, the first portion is designed for remote-field updates and for handling of the complex communication logic with the programming device, in particular with the business layer of the programming device. In an embodiment, the second portion serves for a direct connection to the physical user interface element such that there is no possibility for a software outside the second portion of the patient device to spoof the activation of the physical user interface element. Only upon a real user activation of the physical user interface element, the second portion of the patient device changes a physical modulation to indicate that non-read only commands should be allowed for a predeterminable period of time (that may be a predetermined time during which no reprogramming command has been received by the patient device from the programming device) such as to enable the reprogramming of operational parameters of the implantable medical device. The user activation portion of the user interface may also be part of the second portion of the patient device to minimize its vulnerability to spoofing. In this embodiment, the implantable medical device is specifically designed and arranged to look at the physical link modulation of the communication link between the patient device and the implantable medical device and to provide - depending on characteristics of the modulation - different levels of access to the implantable medical device. These different levels of access typically range from logically read-only access as the lowest access level to full access as the highest access level. All intermediate access levels are conceivable and shall be deemed to be encompassed by the present disclosure.

The predeterminable period of time described above may be for the reprogramming to start. Once started, the non-read only commands may be allowed for a longer time window to give ample time for reprogramming to be completed. However, even after re-programming has begun, it may be important to have some timeout window to turn off the acceptance of non-read only commands, should the reprogramming session be unexpectedly terminated due to, for example, a power blackout at the location of the reprogramming device.

In an embodiment, the second portion comprises at least one of a system for trusted boot and integrity verification and a system for testing whether the second portion is unmodified. These systems enable a particularly safe operation of the patient device and allow security testing of the current integrity of the patient device. Such a testing can be done, e.g., on a periodic basis to continuously monitor the integrity of the patient device and to enhance the security of any reprogramming functionalities of the implantable medical device.

In an embodiment, the programming device, in particular the business layer of the programming device, only allows remote programming to take place on the implantable medical device through the patient device if the patient device has proven that it is unmodified by security attestation, in particular by security attestation performed by any of the systems explained in the preceding paragraph.

In an embodiment, both the patient device and the implantable medical device comprise a communication unit enabling communication between the patient device and the implantable medical device in an inductive manner. This communication unit comprises, in an embodiment, a coil for the near-field communication (also referred to as proximity-based communication) between the patient device and the implantable medical device in an inductive manner. Such an inductive communication link between the patient device and the implantable medical device requires a close proximity of both devices so that a remote attack onto such communication link is extremely difficult or even impossible.

In an embodiment, the remote programming assembly features logging of specific events, in particular a comprehensive audit logging. Examples of events to be logged are the logging of interrogations, the logging of patient consent to remote programming by activating the physical user interaction element, the logging of clinician remote programming attempts, and the logging of administrative actions, in particular all administrative actions.

In an embodiment, the user of the programming device is required to log in the programming device for a time limited access to both view data and perform remote programming of the implantable medical device. In an embodiment, this access to the remote programming device includes an authentication via multifactor authentication (MFA). This increases the security of the overall system since only authenticated personal is able to gain access to the programming device and send out reprogramming commands to the implantable medical device via the patient device.

In an embodiment, the implantable medical device is an implantable pulse generator (IPG), a leadless pacemaker, an implantable cardioverter-defibrillator (ICD), a neuromodulation device, or a device for cardiac resynchronization therapy (CRT).

In an aspect, the present disclosure relates to a method for enabling safe communication between the programming device and an implantable medical device, in particular within a remote programming assembly according to the preceding explanations. This method comprises the steps explained in the following.

In a step, a physical user interface element of the patient device is activated by a user. In a further step, the patient device is transferred, due to the activation of the physical user interface element, from a first state to a second state. In the first state, the patient device only allows an interrogation of an operatively coupled (i.e., associated) implantable medical device. In the second state, the patient device allows also a reprogramming of operational parameters of the implantable medical device. Thus, in its first state, the implantable medical device only provides limited access, wherein, in its second state, it provides enhanced (or even full) access.

In a further method step, a reprogramming command is transmitted from a programming device located remote from the implantable medical device to the patient device. This reprogramming command serves for reprogramming operational parameters of the implantable medical device.

In a further method step, a near-field communication link between the patient device and the implantable medical device is established.

In a further method step, the reprogramming command is transmitted by the patient device to the implantable medical device. This results in a reprogramming of operational parameters of the implantable medical device. If the patient device is not transferred from its first state into its second state, but rather remains in its first state, no reprogramming commands received by the patient device will be transferred to the implantable medical device. This can be realized, e.g., by a denial of transmitting such reprogramming commands by the patient device or by denial of receiving such reprogramming commands by the implantable medical device if the patient device cannot prove to be in its second state.

In an embodiment, the method further comprises the steps of generating an authentication code by the patient device and of transmitting the reprogramming command to the implantable medical device only if the reprogramming command comprises the authentication code. If the reprogramming command does not comprise the authentication code, it is not accepted as legitimate. This embodiment features a defense in depth on top of other security mechanisms in place on the interface between the patient device and the programming device (e.g., the business layer of the programming device) and serves to ensure that the clinician is in direct communication with the patient at the time of the transaction.

In an embodiment, the method further comprises the step of requiring the transmission of the authentication code to the programming device via a second communication link. This second communication link is independent from a first communication link established between the programming device and patient device for transmitting the reprogramming command from the programming device to the patient device. The transmission of the authentication code via the second communication link can be performed, in an embodiment, in a fully automatic way after having established the second communication link. Alternatively, it can be required that the authentication code is provided by the patient to a clinician via a telephone call during the intended reprogramming procedure. In such a case, the authentication code is displayed on the patient device so that the patient can easily read and share it with the clinician being responsible for the reprogramming of the implantable medical device. The programming device then includes this authentication code in the data packs it sends containing the programming commands, and the patient device only forward these programming commands to the implantable medical device if the data packets contain the correct authentication code.

In an embodiment, the method additionally comprises the step of automatically transferring the patient device from the second state into the first state upon expiration of a predeterminable period of time during which no reprogramming command has been received by the patient device from the programming device. This time-out functionality ensures that the patient device does not stay in its second state in which it is susceptible for forwarding reprogramming commands to the implantable medical device. This time-out functionality increases the overall security of the remote programming system in which the presently described method is carried out.

All embodiments of the remote programming assembly and its individual components can be combined in any desired way and can be transferred either individually or in any arbitrary manner to the described method. Likewise, all embodiments of the described method can be combined in any desired way and can be transferred either individually or in any arbitrary manner to the remote programming assembly and its individual components.

The various features and advantages of the present invention may be more readily understood with reference to the following detailed description and the embodiments shown in the drawings. Herein,

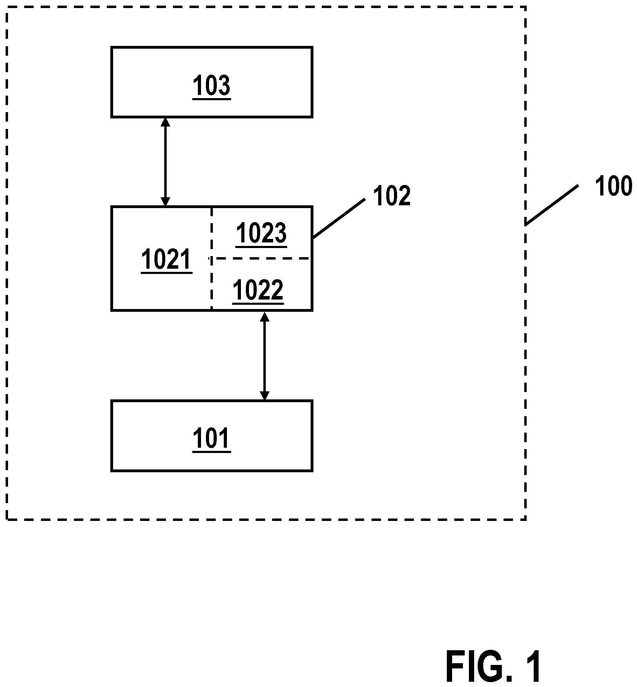

- Fig. 1: schematically illustrates an embodiment of a remote programming assembly and its individual components.

Subsequently, embodiments of the invention shall be described in detail with reference to the drawing. In the drawing, like reference numerals designate like structural elements.

It is to be noted that the embodiments are not limiting for the invention, but merely represent illustrative examples.

Figure 1 schematically depicts an embodiment of a remote programming assembly 100. This remote programming assembly 100 comprises a pacemaker 101 serving as implantable medical device 101. The remote programming assembly 100 further comprises a programming device 103 that is located remote from the pacemaker 101. A patient device 102 is operatively coupled to the pacemaker 101 and to the programming device 103. Any communication between the programming device 103 and the pacemaker 101 is carried out via the patient device 102.

The patient device 102 comprises a first portion 1021 that is updatable. This first portion 1021 is used for establishing a communication between the patient device 102 and the programming device 103. The patient device 102 furthermore comprises a second portion 1022 that is a secured non-updatable portion and that serves for establishing a communication link between the patient device 102 and the implantable medical device 101.

The patient device 102 furthermore comprises a physical user interface element 1023. If a reprogramming of the pacemaker 101 is desired, an according reprogramming command is sent from the programming device 103 to the patient device 102. Before forwarding this reprogramming command from the patient device 102 to the pacemaker 101, the patient device 102 requires an activation of the physical user interface element 1023. Only upon activation of this physical user interface element 1023, the second portion 1022 of the patient device 102 establishes a communication link to the pacemaker 101 and transmits the reprogramming command to the pacemaker 101. In one embodiment the physical user interface element 1023 may be part of the secure non-updatable second portion 1022. This makes it more difficult for a cyberattack to circumvent the physical user interface element 1023.

Expressed in other words, it is always necessary that a user gets into physical interaction with the patient device 102 (namely, via the physical user interface element 1023) to allow a remote programming of the pacemaker 101. This setup enables a remote programming of the pacemaker 101 but secures such remote programming against cyberattacks or other malicious attacks intended to compromise the programming of the pacemaker 101 by a non-authorized third party. Without activation of the physical user interface element 1023, the pacemaker 101 only allows an interrogation by the patient device 102. Even if a malicious attacker gains complete control of the patient device 102, he or she still could not change the state of the pacemaker 101 during a session meant for interrogation only. It is only upon an authorization of the patient or other user by activating the physical user interface element 1023 of the patient device 102 that allows an access to the pacemaker 101 enabling also a reprogramming of operational parameters of the pacemaker 101.

### List of reference numerals

- 100: remote programming assembly
- 101: implantable medical device / pacemaker
- 102: patient device
- 1021: First/updatable portion of the patient device
- 1022: Second/secured non-updateable portion of the patient device
- 1023: physical user interface element of the patient device
- 103: programming device

## Claims

1. A remote programming assembly (100), comprising:
an implantable medical device (101);
a programming device (103) located remote from the implantable medical device (101); and
a patient device (102) operatively connected to the implantable medical device (101) and to the programming device (103);
wherein the patient device (102) and the implantable medical device (101) are designed and configured to communicate via a near-field communication with each other;
wherein the patient device (102) and the implantable medical device (101) are designed and configured to allow a reprogramming of operational parameters of the implantable medical device (101) only upon activation of a physical user interface element (1023) of the patient device (102) by a user.

2. The remote programming assembly (100) according to claim 1, **wherein** the activation of the physical user interface element (1023) can be realized by at least one of reading a fingerprint of the user, entering an authentication key by the user, pushing a button on the patient device (102), facial recognition of the user by a camera of the patient device, and performing a predeterminable physical movement of the patient device (102).

3. The remote programming assembly (100) according to claim 1 or 2, **wherein** the patient device (102) is designed and configured to allow the reprogramming or the start of the reprogramming of the operational parameters of the implantable medical device (101) only during a predeterminable period of time upon activation of the physical user interface element (1023) of the patient device (102).

4. The remote programming assembly (100) according to any of the preceding claims, **wherein** the patient device (102) is designed and configured to generate an authentication code and to forward a reprogramming command for reprogramming operational parameters of the implantable medical device (101) received by the patient device (102) from the programming device (103) to the implantable medical device (101) only if the reprogramming command comprises the authentication code.

5. The remote programming assembly (100) according to claim 4, **wherein** the reprogramming command comprises the authentication code in a cryptographically secured manner.

6. The remote programming assembly (100) according to claim 4 or 5, **wherein** the authentication code has a limited validity period.

7. The remote programming assembly (100) according to any of the preceding claims, **wherein** the patient device (102) is designed and configured to require a multiple-step activation of the physical user interface element (1023) of the patient device (102), wherein a first step of the multiple-step activation serves for generally initiating a reprogramming procedure for the reprogramming of operational parameters of the implantable medical device (101), and wherein a second or subsequent step serves for confirming a specific intended reprogramming command as part of the reprogramming procedure.

8. The remote programming assembly (100) according to any of the preceding claims, **wherein** the patient device (102) comprises an updateable portion (1021) and a secured non-updateable portion (1022), wherein the secured non-updateable portion (1022) serves for establishing a link to the implantable medical device (101) for allowing the reprogramming of operational parameters of the implantable medical device (101) upon activation of the physical user interface element (1023) of the patient device (102).

9. The remote programming assembly (100) according to claim 8, **wherein** the secured non-updateable portion (1022) comprises at least one of a system for trusted boot and integrity verification and a system for attesting that the secured non-updateable portion (1022) is unmodified.

10. The remote programming assembly (100) according to claim 9, **wherein** the programming device (103) is designed and configured to allow a transfer of a reprogramming command for reprogramming operational parameters of the implantable medical device (101) to the patient device (102) only if the patient device (102) provides an attestation that the secured non-updateable portion (1022) is unmodified.

11. The remote programming assembly (100) according to any of the preceding claims, **wherein** the patient device (102) and the implantable medical device (101) each comprise a communication unit enabling a communication between the patient device (102) and the implantable medical device (101) in an inductive manner.

12. A method for enabling safe communication between a programming device (103) and an implantable medical device (101), in particular within a remote programming assembly (100) according to any of the preceding claims, the method comprising the following steps:
activating a physical user interface element (1023) of a patient device (102) by a user;
transferring the patient device (102), by the activation of the physical user interface element (1023), from a first state in which the patient device (102) only allows an interrogation of an operatively coupled implantable medical device (101) to a second state in which the patient device (102) allows also a reprogramming of operational parameters of the implantable medical device (101);
transmitting, from a programming device (103) located remote from the implantable medical device (101) to the patient device (102), a reprogramming command for reprogramming operational parameters of the implantable medical device (101);
establishing a near-field communication between the patient device (102) and the implantable medical device (101); and
transmitting the reprogramming command, by the patient device (102), to the implantable medical device (101) so as to achieve a reprogramming of operational parameters of the implantable medical device (101).

13. The method according to claim 12, further comprising the following steps:
generating, by the patient device (102), an authentication code;
transmitting the reprogramming command to the implantable medical device (101) only if the reprogramming command comprises the authentication code.

14. The method according to claim 13, further comprising the following step:
requiring a transmission of the authentication code to the programming device (103) via a second communication link that is independent from a first communication link established between the programming device (103) and the patient device (102) for transmitting the reprogramming command from the programming device (103) to the patient device (102).

15. The method according to any of claims 12 to 14, further comprising the following step: automatically transferring the patient device (102) from the second state into the first state upon expiration of a predeterminable period of time during which no reprogramming command has been received by the patient device (102) from the programming device (103).
